# EUROPEAN PATENT APPLICATION

(11) **EP 4 807 364 A1**
(43) Date of publication of application: **16.09.2026**
(21) Application number: 24888930.5
(22) Date of filing: 27.09.2024
(51) Int. Cl.: G01N 33/53, G01N 33/543, G01N 33/58, G01N 33/574

(54) **DEVICE AND METHOD FOR AUTOMATED MICRONEEDLE ANALYSIS USING FLUID VORTEX FORMING ELEMENT**

(30) Priority: 08.11.2023 KR 20230153427
(71) Applicant: Kyungpook National University Industry-Academic Cooperation Foundation, Daegu 41566 (KR)
(72) Inventor: HAHN, Young Ki, Seoul 05510 (KR)
(74) Representative: Mewburn Ellis LLP
(86) International application number: PCT/KR2024/014686
(87) International publication number: WO 2025/100735

(57) **Abstract**

The present invention relates to an apparatus for automatically analyzing a microneedle by using a fluid vortex generating element. According to an embodiment of the present invention, an apparatus may include a capturing unit configured to capture a biomarker by being temporarily attached to a part of a body of a subject or being immersed in a sample including the biomarker, and a reaction unit configured to detect a captured biomarker by using a fluid vortex, and further include an external-force providing unit configured to generate and provide external force for generating the fluid vortex.

In this way, according to the present invention, an analysis process may be simplified by automatically performing a buffer reaction and change process for a biomarker captured through a microneedle. Also, the reproducibility may be improved and the analysis time may be reduced while analyzing a biomarker.

## Description

### Technical Field

The present invention relates to an apparatus for automatically analyzing a microneedle by using a fluid vortex generating element and a method of automatically analyzing a microneedle, and more specifically, to an apparatus for automatically analyzing a microneedle by using a fluid vortex generating element that automatically detects and analyzes a biomarker extracted through the microneedle using the fluid vortex generating element and a method of automatically analyzing a microneedle.

### Background Art

A technology for detecting a biomarker based on a microneedle detects the biomarker by causing an exchange phenomenon by immersing the biomarker and an antigen in a buffer solution and causing a reaction between the biomarker and the antigen on one side of the microneedle or outside the device by an experimenter, and has a disadvantage in that reproducibility changes depending on the skill of the experimenter.

Also, a method of detecting a molecular biomarker or a biological biomarker for diagnosis mainly relies on extracting a body fluid from a patient and requires an additional processing step, which takes about a day.

Therefore, a technology for detecting a biomarker present in a subject's body more quickly and precisely is needed.

A background technology of the present invention is described in Korean Patent No. 10-1409610 (announced on June 20, 2014).

### Disclosure of Invention

### Technical Problem

An objective of the present invention is to provide an apparatus and method for automatically analyzing a microneedle by using a fluid vortex generating element that automatically detects and analyzes a biomarker extracted through the microneedle using the fluid vortex generating element.

### Solution to Problem

According to an embodiment of the present invention for solving the technical problems, an apparatus for automatically analyzing a microneedle by using a fluid vortex generating element may include a capturing unit configured to capture a biomarker by being temporarily attached to a part of a body of a subject or being immersed in a sample including the biomarker, and a reaction unit configured to detect a captured biomarker by using a fluid vortex, and further include an external-force providing unit configured to generate and provide external force for generating the fluid vortex.

The capturing unit may include a gasket, the microneedle configured to capture the biomarker by being attached to a part of the body of the subject or being immersed in the sample including the biomarker and having an upper surface coupled to the gasket, and a suspension layer having a space into which the microneedle coupled to the gasket is inserted.

The reaction unit may include a reaction chamber, a channel layer including a plurality of inlet channels through which buffers are introduced into the reaction chamber and an outlet channel through which a reacted buffer is discharged, a fluid vortex generating element inserted into the reaction chamber to generate the fluid vortex, and glass coupled to a lower surface of the channel layer.

The fluid vortex generating element may have a filler structure having a portion protruding in a center of a lower surface of the filler structure, and a rotation speed may change according to external force of the external-force providing unit.

An antibody detecting buffer, a washing buffer, and a fluorescent labeling buffer may be sequentially introduced into the reaction chamber, and the reaction unit may block introduction of the other buffers, perform organic exchange of buffers, remove non-specific binding of a surface of the microneedle, and fluorescently label the biomarker.

According to another embodiment of the present invention, a method of automatically analyzing a microneedle by using a fluid vortex generating element may include a step of capturing a biomarker by being temporarily attached to a part of a body of a subject or being immersed in a sample including a biomarker, a step of generating and providing external force for generating a fluid vortex, and a step of detecting a captured biomarker by using the fluid vortex.

### Advantageous Effects of Invention

In this way, according to the present invention, an analysis process may be simplified by automatically performing a buffer reaction and a change process for a biomarker captured through a microneedle.

Also, the reproducibility and sensitivity may be improved while analyzing a biomarker, and analysis time may be reduced.

### Brief Description of Drawings

FIG. 1 is a structural view of an apparatus for automatically analyzing a microneedle, according to an embodiment of the present invention.
FIG. 2 is a view illustrating a manufactured apparatus for automatically analyzing a microneedle, according to an embodiment of the present invention.
FIG. 3 is a view illustrating an example of detecting a biomarker from a subject and analyzing the biomarker, according to an embodiment of the present invention.
FIG. 4 is a diagram illustrating an example of removing non-specific binding by using a fluid vortex generating element, according to an embodiment of the present invention.
FIG. 5 is an example view illustrating a fluid vortex generating element according to an embodiment of the present invention.
FIG. 6 illustrates views and diagrams of shapes of a fluid vortex generating element according to an embodiment of the present invention and vortex generating ability for each shape.
FIG. 7 illustrates diagrams for non-specific binding removal performance of a fluid vortex generating element in an embodiment of the present invention.
FIG. 8 illustrates views specifically showing a process in which buffer exchange occurs in a reaction unit of an apparatus for automatically analyzing a microneedle, according to an embodiment of the present invention.
FIG. 9 illustrates diagrams showing biomarker quantitative analysis and reproducibility improvement performance of an apparatus for automatically analyzing a microneedle, according to an embodiment of the present invention.
FIG. 10 is a flowchart illustrating a method of manufacturing an apparatus for automatically analyzing a microneedle, according to an embodiment of the present invention.
FIG. 11 is a flowchart of a method of automatically analyzing a microneedle, according to another embodiment of the present invention.
FIG. 12 illustrates views of examples of a process of detecting a biomarker by inserting an apparatus for automatically analyzing a microneedle into a skin-mimicking phantom, according to another embodiment of the present invention.
FIG. 13 illustrates diagrams of biomarker quantitative analysis and reproducibility improvement performance of an apparatus for automatically analyzing a microneedle in a skin-mimicking phantom, according to another embodiment of the present invention.
FIG. 14 illustrates diagrams of biomarker quantitative analysis performance of an apparatus for automatically analyzing a microneedle which reduces biomarker detection time, according to another embodiment of the present invention.
FIG. 15 illustrates views and a diagram of biomarker quantitative performance of an apparatus for automatically analyzing a microneedle using an animal model, according to another embodiment of the present invention.

### Mode for Carrying out the Invention

Hereinafter, preferred embodiments according to the present invention are described in detail with reference to the attached drawings. In this process, thicknesses of lines and sizes of components illustrated in the drawings may be exaggerated for the sake of clarity and convenience of description.

Throughout the specification, when a portion is said to "include" a component, this does not mean that other components are excluded, but rather that, other components may be included therein, unless otherwise specifically stated.

Also, terms described below are terms defined in consideration of functions in the present invention and may change depending on the intention or custom of a user or operator. Therefore, the definitions of the terms should be made based on content throughout the present specification.

In the embodiment described below, the apparatus 100 for automatically analyzing a microneedle uses a magnetic stirrer tool (MST) as a fluid vortex generating element to automatically analyze a microneedle by using a vortex generated by magnetic force, and is described with a specific example. However, the present invention is not limited thereto, and a fluid vortex may be generated through a method, such as magnetic actuation, acoustic actuation, thermal actuation, electrodynamic actuation, installation of a curved and spiral fluid passage and obstacle, or so on.

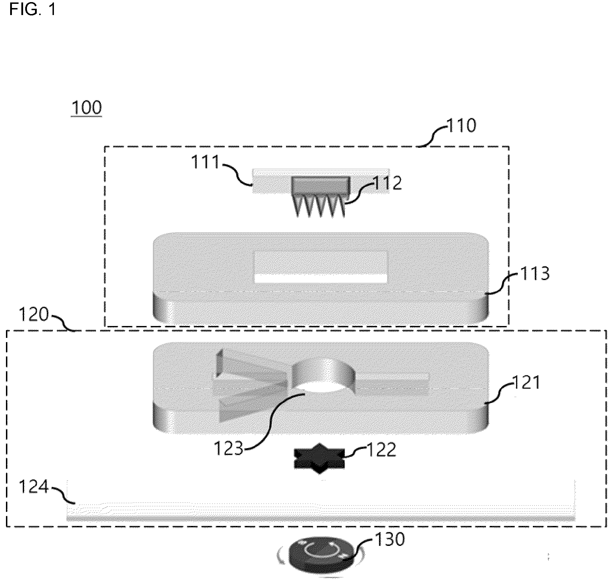

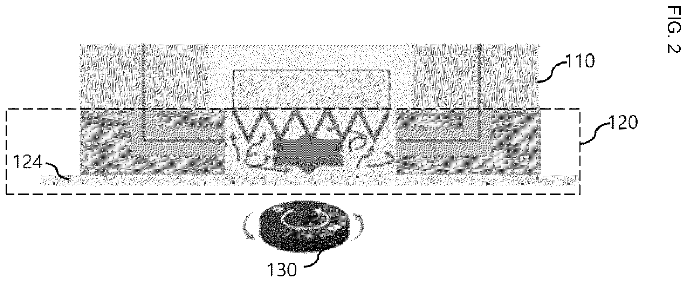

FIG. 1 is a structural view of an apparatus for automatically analyzing a microneedle, according to an embodiment of the present invention, and FIG. 2 is a view illustrating a manufactured apparatus for automatically analyzing a microneedle, according to an embodiment of the present invention.

As illustrated in FIGS. 1 and 2, an apparatus 100 for automatically analyzing a microneedle may include a capturing unit 110, a reaction unit 120, and an external-force providing unit 130.

First, the capturing unit 110 may capture a biomarker by being temporarily attached to a part of a subject's body or immersed in a sample including the biomarker.

Specifically, the capturing unit 110 may include a microneedle 112 that captures a biomarker by being attached to a part of the subject's body or immersed in a sample including the biomarker, and a gasket 111 is coupled to an upper surface of the microneedle 112, and the microneedle 112 coupled to the gasket 111 may be inserted into a suspension layer 113.

Also, the gasket 111 is made of various materials, such as a polymer (Polydimethylsiloxane, PDMS), rubber, silicon, metal, Teflon (Polytetrafluoroethylene, PTFE), ceramic, and so on, and the gasket 111 may be coupled to an upper surface of the microneedle 112. In this case, the gasket 111 may prevent the occurrence of tolerance when the microneedle 112 is inserted into and mounted on the suspension layer 113, and may prevent a sample from leaking.

Next, the microneedle 112 may be composed of various materials, such as, metal, polymer, silicon, natural ingredients, drugs, and so on, and may capture a biomarker by being attached to a part of a subject's body or immersed in a sample including the biomarker.

According to an embodiment, the microneedle 112 may capture a biomarker included in a skin interstitial fluid or sample from a subject. For example, the biomarker may be S100B, which is a melanoma biomarker.

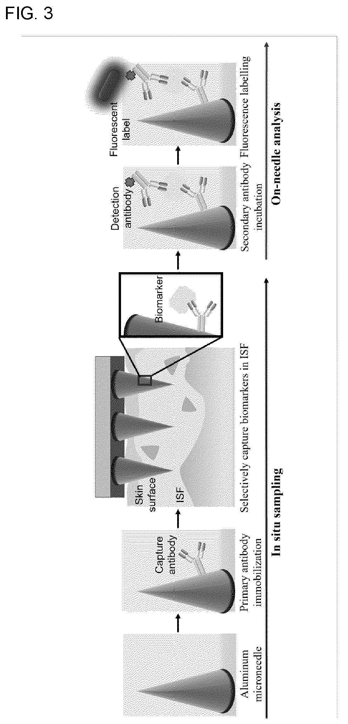

FIG. 3 is a view illustrating an example of detecting a biomarker from a subject and analyzing the biomarker, according to an embodiment of the present invention.

As illustrated in FIG. 3, a primary antibody that reacts with a biomarker may be attached to a surface of the microneedle 112. In this case, the primary antibody may capture a biomarker included in a skin interstitial fluid or sample through an antigen-antibody reaction.

Specifically, a surface of the microneedle 112 may be coated to have positive charges by performing plasma treatment for a preset time (for example, 10 minutes) under the condition of 10 mTorr, 10 sccm, and 100 W (O₂ gas), treating the surface of the microneedle 112 to have negative charges, and then immersing the microneedle 112 in a positively charged material.

Also, a microneedle 112 having positive charges may be immersed in a solution including a primary antibody to electrically attach the primary antibody to the surface of the microneedle 112.

Next, the microneedle 112 may be attached to a part of a subject to capture a biomarker included in an interstitial fluid (ISF).

Also, the microneedle 112 may be immersed in a sample including a biomarker at a concentration of 0.05 *µ*g/mℓ to 160 *µ*g/mℓ to capture the biomarker.

Next, the suspension layer 113 may have a space into which the microneedle 112 may be inserted, and the microneedle 112 coupled to the gasket 111 may be inserted into the space.

Additionally, after the biomarker is captured, the gasket 111 is attached to an upper surface of the microneedle 112, and the microneedle 112 to which the gasket 111 is attached may be coupled to the suspension layer 113. Here, the suspension layer 113 has a space into which the microneedle 112 is inserted, and the microneedle 112 coupled to the gasket 111 may be inserted into the space.

Also, a reaction unit 120 may be coupled to one lower surface of the suspension layer 113.

Next, the reaction unit 120 may detect a biomarker by reacting the captured biomarker with at least one type of buffer through a fluid vortex.

Specifically, the reaction unit 120 is composed of a reaction chamber 123 and a channel layer 121 including a plurality of inlet channels (inlets) through which buffers are introduced into the reaction chamber 123 and an outlet channel (outlets) through which a reacted buffer is discharged, and a fluid vortex generating element (for example, a magnetic stirrer tool) 122 may be inserted into the reaction chamber 123, and glass (slide glass) 124 may be coupled to a lower surface of the channel layer 121. In this case, the microneedle 112 coupled to the suspension layer 113 may be placed at the exact center of the reaction chamber 123.

Also, various buffers may be sequentially introduced into the reaction chamber 123, and the reaction chamber 123 may block the introduction of the other buffers, and perform organic exchange of buffers. In this case, the organic exchange of buffers is described in more detail in the following embodiment.

Also, the fluid vortex generating element 122 may generate a fluid vortex that causes a reaction between a biomarker and a buffer. In this case, a pillar structure may be formed at the center of a lower surface of the fluid vortex generating element 122. Accordingly, a contact surface with the glass 124 may be reduced to minimize frictional force, and the fluid vortex generating element 122 may be rapidly rotated by magnetic force to cause a fluid vortex to be formed.

Also, the reaction chamber 123 may detect a biomarker by using the generated fluid vortex.

Additionally, a buffer including a secondary antibody may be introduced into the reaction chamber 123 through the first inlet channel, and a biomarker may be coupled to the secondary antibody through an antigen-antibody reaction by using the fluid vortex generated by the fluid vortex generating element 122.

Also, a washing buffer may be introduced into the reaction chamber 123 through the second inlet channel, and non-specific binding (NSB) on a surface of the microneedle 112 may be removed through the fluid vortex generated by the fluid vortex generating element 122. In this case, the reaction chamber 123 may remove binding that is not a structure in which the primary antibody, biomarker, and secondary antibody on a surface of the microneedle 112 are sequentially bound, by using the fluid vortex.

Also, a buffer including a fluorescent material (for example, Streptavidin conjugated Texas red buffer) may be introduced into the reaction chamber 123 through a third inlet channel, and a fluorescent material targeting the secondary antibody may be attached to the microneedle 112 by using the fluid vortex generated by the fluid vortex generating element 122.

After the fluorescent material is attached, a washing buffer is once again introduced into the reaction chamber 123 through the second inlet channel, and accordingly, the non-specific binding on the surface of the microneedle 112 may be removed. In this case, the reaction chamber 123 may remove binding that is not a structure in which the primary antibody, biomarker, secondary antibody, and fluorescent material are sequentially bound on the surface of the microneedle 112.

According to an embodiment, the reaction chamber 123 may be formed at a position corresponding to a position of the microneedle 112 bound to the suspension layer 113.

That is, the apparatus 100 for automatically analyzing a microneedle may attach the captured biomarker to the secondary antibody through an antigen-antibody reaction, and attach the captured biomarker to a nano-label that expresses fluorescence by targeting the secondary antibody.

Due to this, the apparatus 100 for automatically analyzing a microneedle may quantitatively analyze a biomarker through fluorescence signal measurement.

Next, the external-force providing unit 130 may generate and provide external force for generating a fluid vortex.

Specifically, the external-force providing unit 130 may provide a rotating magnetic field that generates a fluid vortex by using a rotating magnet.

Here, the rotating magnet may induce rotation of the fluid vortex generating element 122 while rotating in a predetermined direction (for example, counterclockwise or clockwise) to assist generation of a fluid vortex. In this case, a rotation speed of the fluid vortex generating element 122 may change depending on rotation speeds of the rotating magnet.

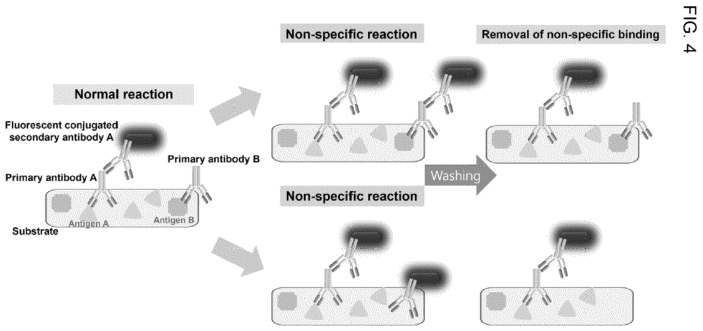

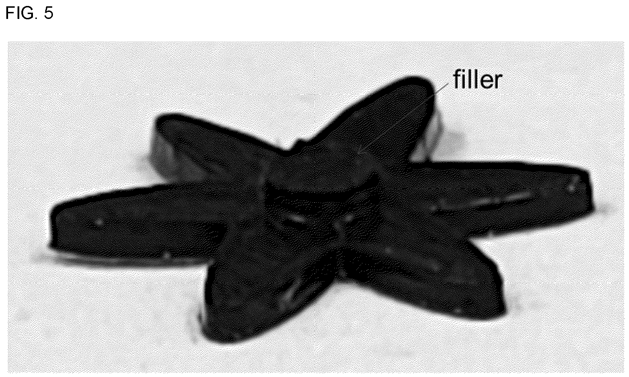

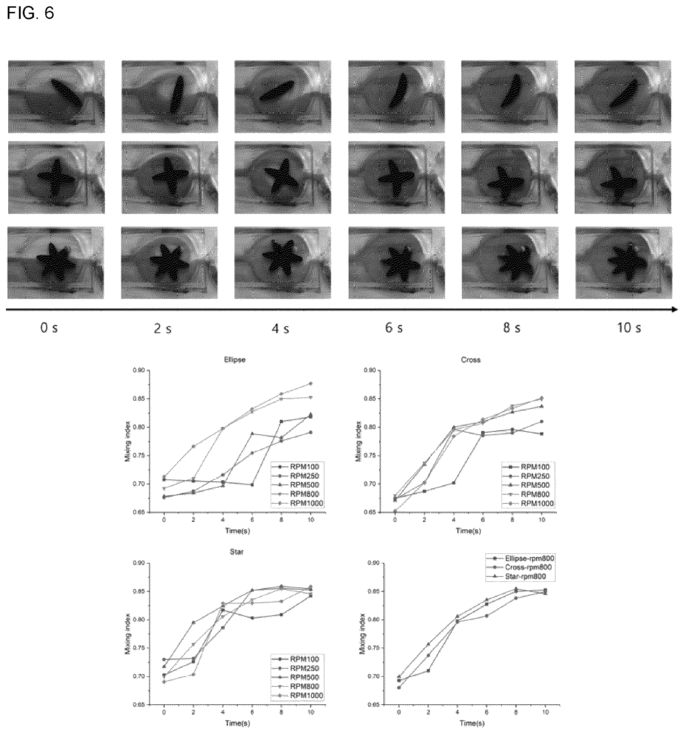

FIG. 4 is a diagram illustrating an example of removing non-specific binding by using a fluid vortex generating element, according to an embodiment of the present invention, FIG. 5 is an example view illustrating a fluid vortex generating element according to an embodiment of the present invention, and FIG. 6 illustrates views and diagrams of shapes of a fluid vortex generating element according to an embodiment of the present invention and vortex generating ability for each shape.

As illustrated in FIG. 4, non-specific binding existing in an immunoassay process may be removed by reacting with a washing buffer by using a fluid vortex generated by the fluid vortex generating element 122 and an external-force providing unit 130.

As illustrated in FIG. 5, the fluid vortex generating element 122 includes a filler having one protruding portion and may be formed in various shapes (for example, an oval shape, a star shape, a cross shape, and so on) that may generate a vortex by friction with the buffer, and the shape is not limited to a specific shape. In this case, the fluid vortex generating element 122 has a size of 5 mm in width, 1 mm in length, and 0.5 mm in height, and may be solidified by mixing iron oxide (Fe₃O₄) with a polymer material at a ratio of 10 wt%.

As illustrated in FIG. 6, when comparing vortex generation capabilities of the fluid vortex generating elements 122 having various shapes with each other, it can be seen that sufficient fluid vortex is generated in each buffer in about 8 seconds.

Also, it can be seen that a shape of the fluid vortex generating element 122 causing more resistance generates more vortex in a shorter time, and accordingly, non-specific binding of the microneedle 112 may be efficiently removed in a shorter time.

According to an embodiment, the fluid vortex generating element 122 may generate a fluid vortex by rotating under the influence of a rotating magnetic field of the external-force providing unit 130 in the reaction chamber 123, and may remove non-specific binding through effective washing due to a vortex of the washing buffer.

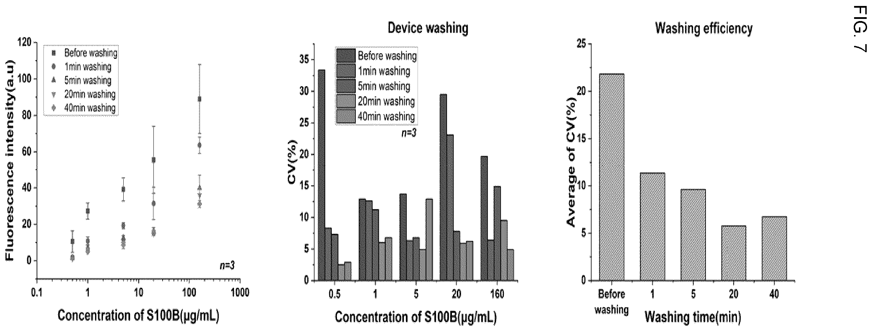

FIG. 7 illustrates diagrams for non-specific binding removal performance of a fluid vortex generating element in an embodiment of the present invention.

Referring to FIG. 7, it can be seen that non-specific binding is effectively removed by performing the washing by using a vortex of a washing buffer generated by rotation of the fluid vortex generating element 122, and as a result, a deviation is greatly reduced along with a decrease in fluorescence signal value, and reproducibility is improved. Also, the fluid vortex generating element 122 may greatly remove non-specific binding by generating a fluid vortex at 800 RPM for 20 minutes.

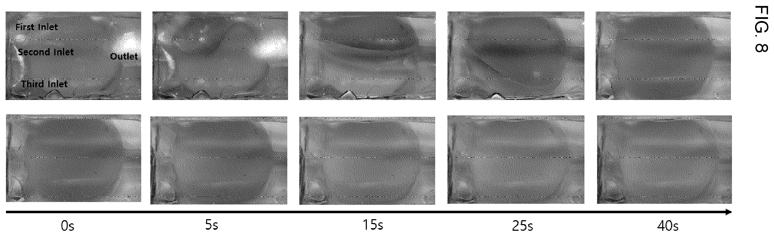

FIG. 8 illustrates views specifically illustrating a process in which buffer exchange occurs in a reaction unit of an apparatus for automatically analyzing a microneedle, according to an embodiment of the present invention.

As illustrated in FIG. 8, buffers of three types (an antibody detecting buffer (buffer of detection antibody), a washing buffer, and a fluorescence labeling buffer) may sequentially introduce into the reaction chamber 123 through the first inlet channel (Inlet 1) to a third inlet channel (Inlet 3).

Also, the reaction chamber 123 may include a plurality of inlet channels (inlet 1, ..., inlet n) and one outlet channel (an outlet). Here, buffers may sequentially introduce into the reaction chamber 123 through the plurality of inlet channels, and the reaction chamber 123 may block the introduction of the other buffers, perform organic exchange of buffers, removes non-specific binding on ae surface of the microneedle 112, and fluorescently label a biomarker.

In other words, organic exchange of the antibody detecting buffer, the washing buffer, and the fluorescent labeling buffer occurs in the reaction chamber 123, and the reaction chamber 123 may remove non-specific binding that may exist on a surface of the microneedle 112 through a fluid vortex generated by the fluid vortex generating element 122 and detect a biomarker by fluorescently labeling the biomarker.

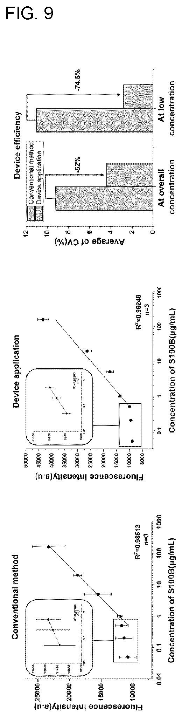

FIG. 9 illustrates diagrams showing biomarker quantitative analysis and reproducibility improvement performance of an apparatus for automatically analyzing a microneedle, according to an embodiment of the present invention.

Referring to FIG. 9, when comparing a case where a biomarker is detected by reacting multiple buffers with each other and immersing the microneedle 112 in a buffer by an experimenter without using the apparatus 100 for automatically analyzing a microneedle (conventional method) with a case where a biomarker is detected by using the apparatus 100 for automatically analyzing a microneedle (device application), the case where the biomarker is detected by using the apparatus 100 for automatically analyzing a microneedle shows a less deviation and an improvement in reproducibility of about 52%, and a greater improvement of 74.5% in reproducibility is obtained in a lower biomarker concentration range.

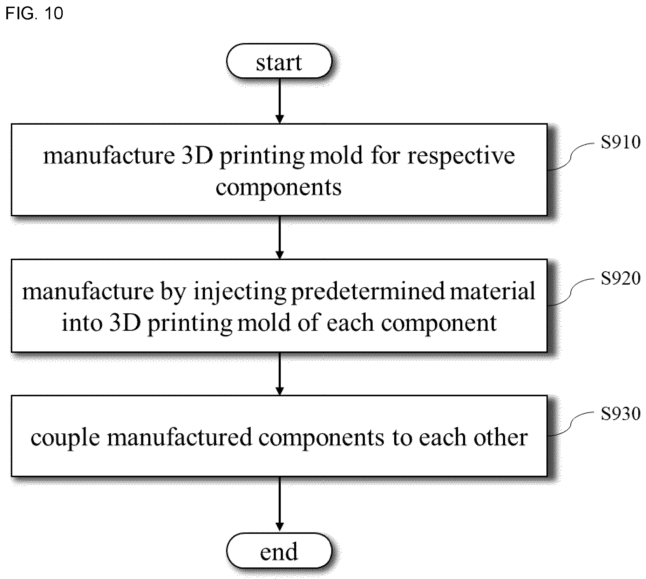

FIG. 10 is a flowchart illustrating a method for manufacturing the apparatus for automatically analyzing a microneedle, according to an embodiment of the present invention.

As illustrated in FIG. 10, the apparatus 100 for automatically analyzing a microneedle may be manufactured through step S910 of manufacturing a 3D printing mold for respective components of the capturing unit 110 and the reaction unit 120 by using a 3D printer, step S920 of injecting predetermined materials into 3D printing molds of the respective components, and step S930 of coupling the manufactured components to each other. In this case, the 3D printing molds may each be manufactured according to a predetermined drawing.

Here, the microneedle 112 may be manufactured by using a conventional manufacturing method or manufactured, through a milling process, by being digitized by a computer numerical controller in a shape of a bullet, which has a bottom diameter of 0.25 mm and a height of 0.7 mm.

In this case, the suspension layer 113 or the reaction chamber 123 may be manufactured through a method of injecting a predetermined material up to a predetermined height of the 3D printing mold and solidifying the predetermined material for being bound to the microneedle 112 or the fluid vortex generating element 122. In this case, an embossed portion is directed toward a bottom surface.

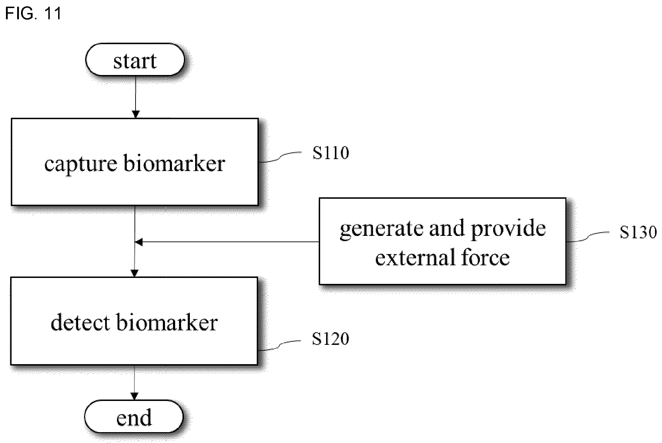

FIG. 11 is a flowchart of a method of automatically analyzing a microneedle, according to another embodiment of the present invention.

As illustrated in FIG. 11, the capturing unit 110 may capture a biomarker by being temporarily attached to a part of a subject's body or immersed in a sample including the biomarker (S110).

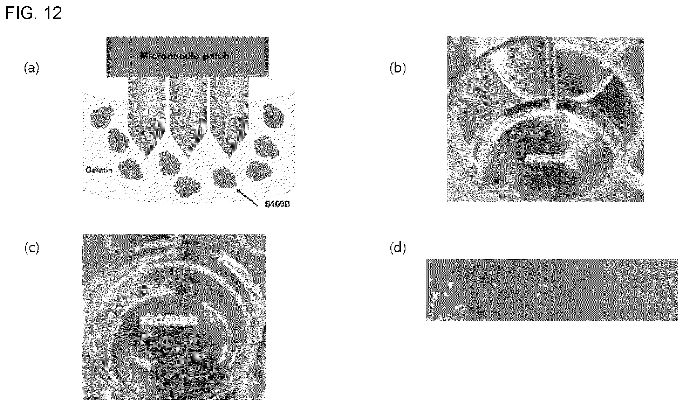

FIG. 12 illustrates views of examples of a process of detecting a biomarker by inserting an apparatus for automatically analyzing a microneedle into a skin-mimicking phantom, according to another embodiment of the present invention.

FIG. 12 (a) is a view illustrating an example of inserting the apparatus 100 for automatically analyzing a microneedle into a skin-mimicking phantom, FIG. 12 (b) is a shape viewed from above of the apparatus 100 for automatically analyzing a microneedle inserted into the skin-mimicking phantom, and FIG. 12 (c) is a shape viewed from below of the apparatus 100 for automatically analyzing a microneedle inserted into the skin-mimicking phantom.

As illustrated in FIG. 12 (a) to FIG. 12 (c), the capturing unit 110 may detect a biomarker existing in the skin-mimicking phantom by being inserted into the skin-mimicking phantom. As illustrated in FIG. 12 (d), when the apparatus 100 for automatically analyzing a microneedle inserted into the skin-mimicking phantom is removed, it can be seen that no damage occurs in other portions except for a portion where the apparatus 100 for automatically analyzing a microneedle is inserted.

Thereafter, the reaction unit 120 may detect the captured biomarker by using the fluid vortex (S120).

To this end, the external-force providing unit 130 may generate and provide external force to generate a fluid vortex (S130).

The specific operations performed in respective steps are described with reference to FIGS. 1 to 9 above, and accordingly, redundant descriptions thereof are omitted.

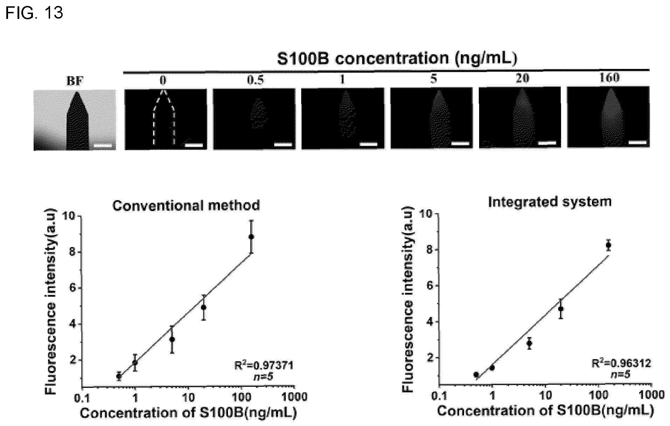

FIG. 13 illustrates diagrams of biomarker quantitative analysis and reproducibility improvement performance of an apparatus for automatically analyzing a microneedle in a skin-mimicking phantom, according to another embodiment of the present invention.

As illustrated in FIG. 13, when comparing a case where a biomarker is detected by reacting multiple buffers with a skin-mimicking phantom and immersing the microneedle 112 in a buffer by an experimenter without using the apparatus 100 for automatically analyzing a microneedle (a conventional method) with a case where the biomarker is detected by using the apparatus 100 for automatically analyzing a microneedle (an integrated system), it can be seen that the case where the biomarker is detected by using the apparatus 100 for automatically analyzing a microneedle shows a lower detection limit with less deviation.

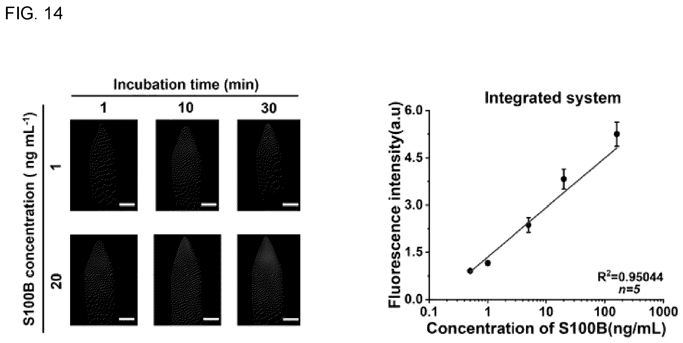

FIG. 14 illustrates diagrams of biomarker quantitative analysis performance of an apparatus for automatically analyzing a microneedle which reduces biomarker detection time, according to another embodiment of the present invention.

As illustrated in FIG. 14, it can be seen that the apparatus 100 for automatically analyzing a microneedle may quantitatively analyze a biomarker while reducing biomarker detection time from 30 minutes to 10 minutes and 1 minute and has high reproducibility.

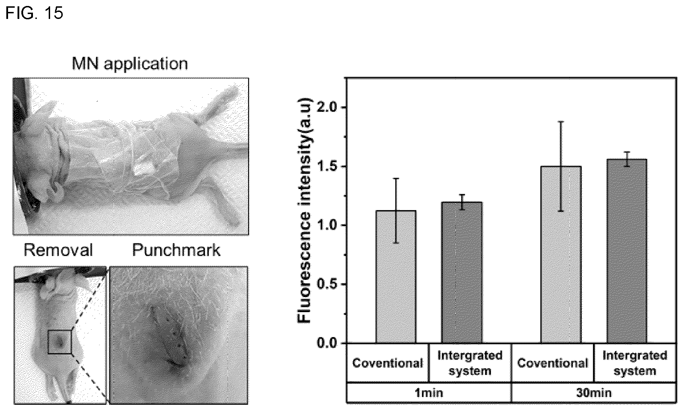

FIG. 15 illustrates views and a diagram of biomarker quantitative performance of an apparatus for automatically analyzing a microneedle using an animal model, according to another embodiment of the present invention.

As illustrated in FIG. 15, when a biomarker is detected from an animal model having skin melanoma by using the apparatus 100 for automatically analyzing a microneedle, it can be seen that the biomarker may be detected within 30 minutes as illustrated in FIG. 14 and the biomarker may be detected with improved reproducibility even with a detection time of 1 minute.

According to another embodiment of the present invention, when a vortex is generated by using an acoustic operation, the fluid vortex generating element 122 may generate acoustic streaming and a fluid vortex through acoustic vibration by using a translator. In this case, the translator may be embedded in a piezoelectric substrate, and the external-force providing unit 130 may provide surface acoustic waves (SAW) (for example, Rayleigh waves) that generate a fluid vortex.

Here, the fluid vortex generating element 122 may generate a fluid vortex by using energy leaking at a Rayleigh wave angle.

According to another embodiment of the present invention, when a vortex is generated by using a thermal operation, the fluid vortex generating element 122 may generate a fluid vortex through a density change and a Marangoni flow. In this case, the external-force providing unit 130 may include a small heater and form a temperature gradient by locally heating a part of the reaction unit 120, and as the temperature of the locally heated part of the reaction unit 120 increases, a surface tension decreases, and a surface tension gradient may be generated.

Here, the fluid vortex generating element 122 may generate a fluid vortex by using a surface tension gradient that promotes a fluid flow along a boundary from a hot region to a cold region.

According to another embodiment of the present invention, when a vortex is generated by using an electrodynamic operation, the fluid vortex generating element 122 may generate a fluid vortex by manipulating a fluid and particles through an electric field, and the reaction chamber 123 may include an electrolyte solution. In this case, the external-force providing unit 130 may apply an alternating current (AC) electric field to an electrode in contact with the electrolyte solution in the reaction chamber 123 or provide an electric field.

Here, when a vortex is generated through AC electroosmosis, the external-force providing unit 130 may apply an AC electric field to an electrode in contact with the electrolyte solution, and the fluid vortex generating element 122 may induce charges in a thin electric double layer near an electrode surface formed by the applied AC electric field and may generate a fluid vortex according to an interaction between the induced charges and a tangential component of the AC electric field. In this case, a fluid speed may change depending on frequencies, and the fluid vortex generating element 122 may generate fluid pumping and a fluid vortex according to an electrode pattern design.

Also, when a vortex is generated through induced charge electroosmosis, the external-force providing unit 130 may provide an electric field, the fluid vortex generating element 122 may induce charges on a polarizable surface according to the electric field, the induced charge may attract counter ions to form an induced electric double layer, and a fluid vortex may be generated by an interaction between the induced electric double layer and the applied electric field.

In this case, the fluid vortex generating element 122 may perform manipulation of particles and a fluid according to the design of a metal post array inside a microchannel.

According to another embodiment of the present invention, when a vortex is generated by using a curved and spiral fluid passage, the apparatus 100 for automatically analyzing a microneedle may not include the external-force providing unit 130, the reaction unit 120 may have a curved or spiral microchannel, and the fluid vortex generating element 122 may generate a Dean vortex by using centrifugal force.

In this case, when a fluid passes through a curved channel, a pressure gradient may be generated through a difference in fluid velocity at the center higher than at a boundary of the reaction chamber 123 due to the centrifugal force and a no-slip condition, and a secondary flow may be induced through the pressure gradient, and accordingly, the fluid vortex generating element 122 may generate a fluid vortex.

Also, the reaction unit 120 may efficiently perform particle manipulation or generate a fluid vortex by adjusting an aspect ratio, a curvature radius, and so on of the reaction chamber 123.

According to another embodiment of the present invention, when a vortex is generated by installing an obstacle, the apparatus 100 for automatically analyzing a microneedle may not include the external-force providing unit 130, and the fluid vortex generating element 122 may generate a fluid vortex by being provided as an obstacle in the reaction chamber 123.

In this case, the fluid vortex generating element 122 may generate a fluid vortex by causing a change in height of the reaction chamber 123 to locally induce a pressure gradient and flow separation, or may generate a fluid vortex by being placed in a path through which a fluid flows in the form of a micro-pillar, or may generate a transverse or spiral fluid vortex according to a width, a depth, and so on of a groove of a structure by being placed on the bottom of the reaction chamber 123 in a preset structure (for example, a herringbone structure).

According to an embodiment of the present invention, an analysis process may be simplified by automatically performing a buffer reaction and change process for a biomarker captured through a microneedle.

Also, the reproducibility may be improved and the analysis time may be reduced while analyzing a biomarker.

Although the present invention is described with reference to the embodiments illustrated in the drawings, the embodiments are merely examples, and those skilled in the art to which the present invention belongs will understand that various modifications and equivalent other embodiments may be derived therefrom. Therefore, the true technical protection scope of the present invention should be determined by the technical idea of the following patent claims.

### Signs List

| | | | |
|---|---|---|---|
| 100: | apparatus for automatically analyzing microneedle | | |
| 110: | capturing unit | | |
| 111: | gasket | 112: | microneedle |
| 113: | suspension layer | | |
| 120: | reaction unit | | |
| 121: | channel layer | 122: | fluid vortex generating element |
| 123: | reaction chamber | 124: | glass |
| 130: | external-force providing unit | | |

## Claims

1. An apparatus for automatically analyzing a microneedle by using a fluid vortex generating element, the apparatus comprising:
a capturing unit configured to capture a biomarker by being temporarily attached to a part of a body of a subject or being immersed in a sample including the biomarker; and
a reaction unit configured to detect a captured biomarker by using a fluid vortex.

2. The apparatus for automatically analyzing the microneedle of claim 1, further comprising:
an external-force providing unit configured to generate and provide external force for generating the fluid vortex.

3. The apparatus for automatically analyzing the microneedle of claim 1, wherein the capturing unit includes:
a gasket;
the microneedle configured to capture the biomarker by being attached to a part of the body of the subject or being immersed in the sample including the biomarker and having an upper surface coupled to the gasket; and
a suspension layer having a space into which the microneedle coupled to the gasket is inserted.

4. The apparatus for automatically analyzing the microneedle of claim 1, wherein the reaction unit includes:
a reaction chamber;
a channel layer including a plurality of inlet channels through which buffers are introduced into the reaction chamber and an outlet channel through which a reacted buffer is discharged;
a fluid vortex generating element inserted into the reaction chamber to generate the fluid vortex; and
glass coupled to a lower surface of the channel layer.

5. The apparatus for automatically analyzing the microneedle of claim 4, wherein
the fluid vortex generating element has a filler structure having a portion protruding in a center of a lower surface of the filler structure, and a rotation speed changes according to external force of the external-force providing unit.

6. The apparatus for automatically analyzing the microneedle of claim 4, wherein
an antibody detecting buffer, a washing buffer, and a fluorescent labeling buffer are sequentially introduced into the reaction chamber, and the reaction unit blocks introduction of the other buffers, performs organic exchange of buffers, removes non-specific binding of a surface of the microneedle, and fluorescently labels the biomarker.

7. A method of automatically analyzing a microneedle, the method comprising:
a step of capturing a biomarker by being temporarily attached to a part of a body of a subject or being immersed in a sample including a biomarker; and
a step of detecting a captured biomarker by using a fluid vortex.

8. The method of automatically analyzing the microneedle of claim 7, further comprising:
a step of generating and providing external force for generating the fluid vortex.

9. The method of automatically analyzing the microneedle of claim 7, wherein,
in the detecting step, an antibody detecting buffer, a washing buffer, and a fluorescent labeling buffer are sequentially introduced into the reaction chamber, and the reaction unit blocks introduction of the other buffers, performs organic exchange of buffers, removes non-specific binding of a surface of the microneedle, and fluorescently labels the biomarker.
